(19) Europäisches Patentamt
European Patent Office
Office européen des brevets

(11) Veröffentlichungsnummer: **0 384 314 A2**

# (12) EUROPÄISCHE PATENTANMELDUNG

(21) Anmeldenummer: **90103020.5**

(22) Anmeldetag: **16.02.90**

(51) Int. Cl.5: **C07D 333/38, C07D 333/44, C07D 409/12, C07D 409/06, C07D 417/06**

(30) Priorität: **23.02.89 DE 3905577**

(43) Veröffentlichungstag der Anmeldung: **29.08.90 Patentblatt 90/35**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Wiesenfeldt, Matthias, Dr.**
**Rosenstrasse 10**
**D-6704 Mutterstadt(DE)**
Erfinder: **Etzbach, Karl-Heinz, Dr.**
**Carl-Bosch-Ring 55**
**D-6710 Frankenthal(DE)**

## (54) Diaminothiophene.

(57) Diaminothiophene der Formel

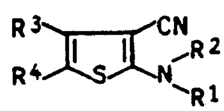

oder deren Tautomere, worin
$R^1$ und $R^2$ jeweils Wasserstoff oder zusammen einen Rest der Formel

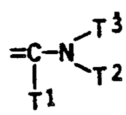

,

in der $T^1$ für Wasserstoff, Alkyl oder Phenyl, $T^2$ und $T^3$ unabhängig voneinander für Alkyl oder Phenyl, oder $T^2$ und $T^3$ zusammen mit dem sie verbindenden Stickstoffatom für einen heterocyclischen Rest stehen,
$R^3$ substituiertes Amino und
$R^4$ Alkanoyl, Benzoyl, Cyano, Nitro oder ein Rest der Formel

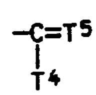

bedeuten, worin $T^4$ für Wasserstoff, Alkyl oder Phenyl und $T^5$ für den Rest eines primären Amins oder einer methylenaktiven Verbindung stehen,
sowie ein Verfahren zu ihrer Herstellung.

EP 0 384 314 A2

## Diaminothiophene

Die vorliegende Erfindung betrifft neue Diaminothiophene der Formel I

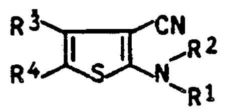

(I)

oder deren Tautomere, wobei

$R^1$ und $R^2$ jeweils Wasserstoff oder zusammen einen Rest der Formel

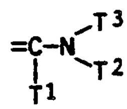

,

worin $T^1$ für Wasserstoff, $C_1$-$C_4$-Alkyl oder Phenyl und $T^2$ und $T^3$ gleich oder verschieden sind und unabhängig voneinander jeweils für $C_1$-$C_4$-Alkyl, $C_5$-$C_7$-Cycloalkyl oder Phenyl oder $T^2$ und $T^3$ zusammen mit dem sie verbindenden Stickstoffatom für einen 5- bis 7-gliedrigen gesättigten heterocyclischen Rest, der gegebenenfalls weitere Heteroatome enthält, stehen,

$R^3$ $C_1$-$C_{20}$-Mono- oder Dialkylamino, $C_2$-$C_{10}$-Mono- oder Dialkylamino, worin die Alkylkette substituiert und/oder durch ein oder mehrere Sauerstoffatome unterbrochen ist, $C_3$-$C_8$-Cycloalkylamino, Adamantylamino, $C_2$-$C_{12}$-Mono- oder Dialkenylamino, $C_3$-$C_{12}$-Alkinylamino, N-($C_1$-$C_5$-Alkyl)-N-phenylamino, Pyrrolidino, Piperidino, Morpholino, Thiomorpholino, Piperazino, N-($C_1$-$C_4$-Alkyl)piperazino, Hexamethylenimino, Imidazol-1-yl, Pyrazol-1-yl, gegebenenfalls substituiertes Phenylamino, Pyridylamino, Thienylamino, Hydrazino, $C_1$-$C_4$-Mono- oder Dialkylhydrazino oder Phenylhydrazino und

$R^4$ $C_1$-$C_6$-Alkanoyl, Benzoyl, Cyano, Nitro oder einen Rest der Formel

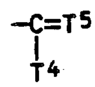

bedeuten, worin $T^4$ für Wasserstoff, $C_1$-$C_4$-Alkyl oder Phenyl und $T^5$ für den Rest einer methylenaktiven Verbindung, Hydroxyimino oder den Rest N-X stehen, in dem X die Bedeutung von $C_1$-$C_{20}$-Alkyl, das gegebenenfalls substituiert und/oder durch ein oder mehrere Sauerstoffatome unterbrochen ist, gegebenenfalls substituiertem $C_3$-$C_6$-Alkenyl, gegebenenfalls substituiertem $C_3$-$C_6$-Alkinyl, gegebenenfalls substi tuiertem $C_3$-$C_{10}$-Cycloalkyl, gegebenenfalls substituiertem Phenyl, Pyridyl, $C_1$-$C_4$-Alkoxycarbonylmethyl, Amino, $C_1$-$C_4$-Dialkylamino oder Phenylamino besitzt.

Es sind bereits 2,4-Diaminothiophenderivate bekannt, die in Ringposition 2 eine substituierte Aminogruppe und in Ringposition 4 eine unsubstituierte Aminogruppe aufweisen (siehe z.B. J. Prakt. Chem., Band 326, S. 459 bis 464, 1986; J.Chem.Soc. Perkin Trans.I, 1986, S. 1171 bis 1179 oder Monatsh. Chem. Band 112, S. 1393 bis 1404, 1981). In der CH-A-583 224 wird weiterhin die Herstellung von 2,4-Diamino-3,5-dicyanothiophen beschrieben. Die genannten Diaminothiophene eignen sich jedoch nicht besonders gut als Diazokomponenten für die Herstellung von Azofarbstoffen.

Aufgabe der vorliegenden Erfindung war es nun, neue Diaminothiophene bereitzustellen, die in Ringposition 2 eine unsubstituierte Aminogruppe und in Ringposition 4 eine substituierte Aminogruppe aufweisen sollten.

Demgemäß wurden die oben näher bezeichneten Diaminothiophene der Formel I gefunden.

Alle in der obengenannten Formel I auftretenden Alkyl- und Alkenylgruppen können sowohl geradkettig als auch verzweigt sein.

Wenn in der Formel I Alkylgruppen auftreten, die durch ein oder mehrere Sauerstoffatome unterbrochen sind, sind jene bevorzugt, die durch ein bis drei, insbesondere ein bis zwei Sauerstoffatome unterbrochen sind.

Wenn in der Formel I substituierte Phenylgruppen auftreten, kommen als Substituenten z.B. $C_1$-$C_4$-Alkyl, $C_1$-$C_4$-Alkoxy, Halogen, dabei insbesondere Chlor oder Brom, Nitro, Hydroxy, Amino, $C_1$-$C_4$-Dialkylamino, Carboxyl oder $C_1$-$C_4$-Alkanoyl in Betracht.

Wenn in der Formel I substituierte Alkylgruppen auftreten, kommen als Substituenten z.B. $C_1$-$C_5$-Alkylthio, gegebenenfalls substituiertes Phenoxy, Halogen, dabei insbesondere Chlor oder Brom, Hydroxy, Amino, $C_1$-$C_4$-Mono-oder Dialkylamino, Pyrrolidino, Piperidino, Morpholino, Thiomorpholino, Piperazino, N-($C_1$-$C_4$-Alkyl)piperazino, $C_1$-$C_5$-Alkoxycarbonyl oder gegebenenfalls substituiertes Phenyl in Betracht.

Wenn in der Formel I substituiertes Alkenyl-, Alkinyl- oder Cycloalkylgruppen auftreten, kommen als Substituenten z.B. Fluor, Chlor oder Brom in Betracht.

Wenn die Reste $T^2$ und $T^3$ zusammen mit dem sie verbindenden Stickstoffatom einen 5- bis 7-gliedrigen gesättigten heterocyclischen Rest, der gegebenenfalls weitere Heteroatome enthält, bedeuten, so kommen dafür z.B. Pyrrolidino, Piperidino, Morpholino, Thiomorpholino, Thiomorpholino-S,S-dioxid, Piperazino, N-($C_1$-$C_4$-Alkyl)piperazino oder Hexamethylenimino in Betracht.

Reste $T^1$, $T^2$, $T^3$ und $T^4$ sind beispielsweise Methyl, Ethyl, Propyl, Isopropyl, Butyl, Isobutyl oder sec-Butyl.

Reste $R^4$ sind beispielsweise Formyl, Acetyl, Propionyl, Butyryl, Isobutyryl, Pentanoyl oder Hexanoyl.

(Die im folgenden verwendeten Bezeichnungen Isooctyl, Isononyl, Isodecyl und Isotridecyl sind Trivialbezeichnungen und stammen von den nach der Oxosynthese erhaltenen Alkoholen (vgl. dazu Ullmanns Encyklopädie der technischen Chemie, 4. Auflage, Band 7, Seiten 215 bis 217, sowie Band 11, Seiten 435 und 436.)

Der Rest -NX leitet sich von primären Aminen der Formel $H_2NX$ ab. Als solche sind beispielsweise Methylamin, Ethylamin, Propylamin, Isopropylamin, Butylamin, Isobutylamin, sec-Butylamin, tert-Butylamin, Pentylamin, Isopentylamin, Neopentylamin, Hexylamin, Heptylamin, n-Octylamin, Isooctylamin, 2-Ethylhexylamin, Nonylamin, Isononylamin, Decylamin, Isodecylamin, Undecylamin, Dodecylamin, Tridecylamin, Isotridecylamin, Tetradecylamin, Pentadecylamin, Hexadecylamin, Heptadecylamin, Octadecylamin, Nonadecylamin, Eicosylamin, Allylamin, Methallylamin, Propargylamin, Cyclopropylamin, Cyclobutylamin, Cyclopentylamin, Cyclohexylamin, Cycloheptylamin, Cyclooctylamin, Cyclononylamin, Cyclodecylamin, 2-Hydroxyethylamin, 2-Methoxyethylamin, 2-Ethoxyethylamin, 3-Hydroxypropylamin, 3-Methoxypropylamin, 3-Ethoxypropylamin, 3-(2-Phenoxyethoxy)propylamin, 3-Benzyloxypropylamin, 2-(N,N-Dimethylamino)-ethylamin, 2-(N,N-Diethylamino)ethylamin, 3-(N,N-Dimethylamino)propylamin, 3-(N,N-Diethylamino)-propylamin, Benzylamin, 2-Phenylethylamin, 3-Phenylpropylamin, Anilin, 2-Hydroxyanilin, 3-Hydroxyanilin, 4-Hydroxyanilin, o-Anisidin, m-Anisidin, p-Anisidin, o-Phenetidin, m-Phenetidin, p-Phenetidin, 2-Chloranilin, 3-Chloranilin, 3-Nitroanilin, 4-Nitroanilin, o-Toluidin, m-Toluidin, p-Toluidin, 1,2-Phenylendiamin, 1,3-Phenylendiamin, 1,4-Phenylendiamin, 2-Ethylanilin, 3-Ethylanilin, 4-Ethylanilin, 2-Aminopyridin, 3-Aminopyridin, 4-Aminopyridin, Glycinmethylester, Glycinethylester, Glycinpropylester, Glycinbutylester, Hydrazin, N,N-Dimethylhydrazin oder Phenylhydrazin zu nennen.

Der Rest $T^5$ leitet sich z.B. von methylenaktiven Verbindungen der Formel $H_2T^5$ ab. Solche Verbindungen gehorchen beispielsweise der Formel

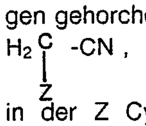

in der Z Cyano, Nitro, $C_1$-$C_6$-Alkanoyl, Benzoyl, $C_1$-$C_4$-Alkylsulfonyl, Phenylsulfonyl, Carboxyl, $C_1$-$C_4$-Alkoxycarbonyl, Carbamoyl oder $C_1$-$C_4$-Mono- oder Dialkylcarbamoyl bedeutet, oder der Formel

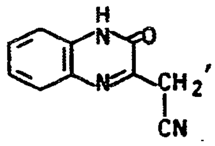, 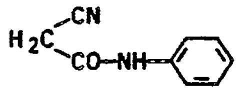, 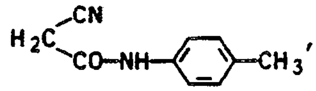, 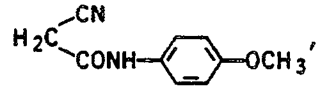,

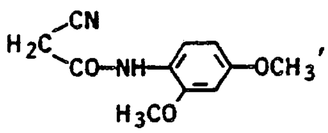, 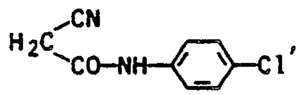, 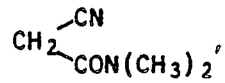, 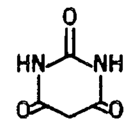,

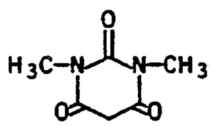, 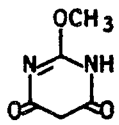, 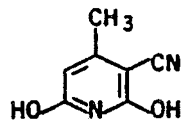, 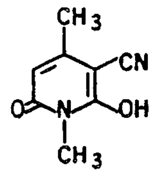, 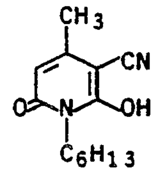,

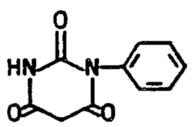, 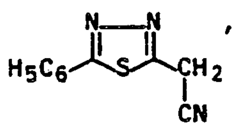, 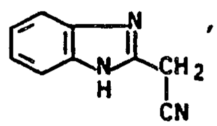, 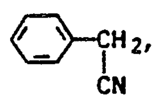, 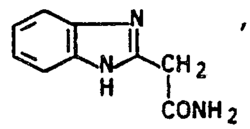,

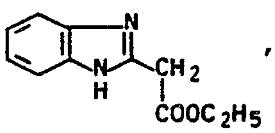, 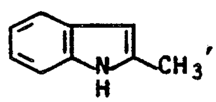, 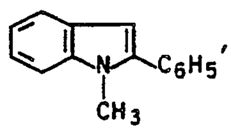, 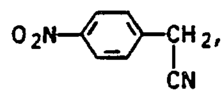,

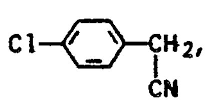, 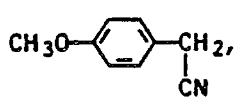, 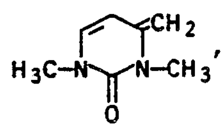, $CH_3-NO_2$, $C_2H_5-NO_2$,

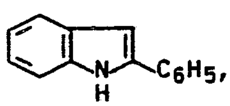, 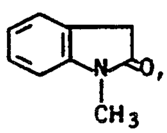, 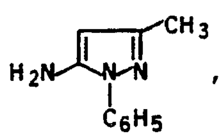, 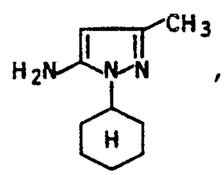, 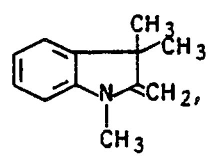,

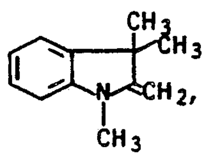, 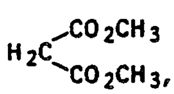, 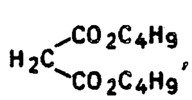, 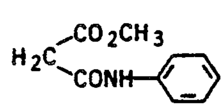 oder 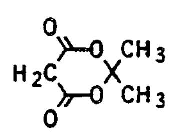

Einzelne besonders wichtige Verbindungen sind z.B.:

$H_2C(CN)_2$, 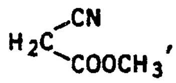, 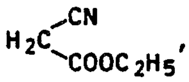, 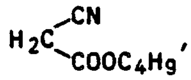, 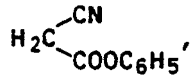, 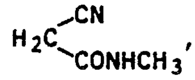,

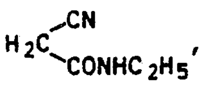, 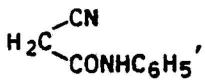, 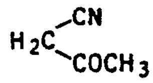 oder 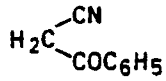 sowie 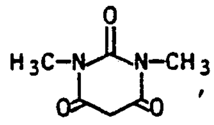,

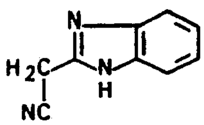, 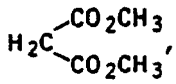, 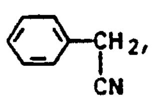, 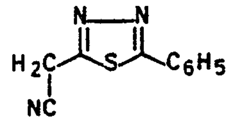 oder 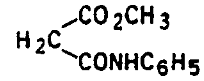.

Reste $R^3$ sind beispielsweise Methylamino, Ethylamino, Propylamino, Isopropylamino, Butylamino, Isobutylamino, sec-Butylamino, Pentylamino, Isopentylamino, Neopentylamino, Hexylamino, Heptylamino, Octylamino, Isooctylamino, 2-Ethylhexylamino, Nonylamino, Isononylamino, Decylamino, Isodecylamino, Undecylamino, Dodecylamino, Tridecylamino, Isotridecylamino, Tetradecylamino, Pentadecylamino, Hexadecylamino, Heptadecylamino, Octadecylamino, Nonadecylamino, Eicosylamino, Allylamino, Propargylamino, Methallylamino, Cyclopropylamino, Cyclobutylamino, Cyclopentylamino, Cyclohexylamino, Cycloheptylamino, Cyclooctylamino, Cyclononylamino, Cyclodecylamino, 2-Hydroxyethylamino, 2-Methoxyethylamino, 2-Ethoxyethylamino, 2-Benzyloxyethylamino, 3-Hydroxypropylamino, 3-Methoxypropylamino, 3-Ethoxypropylamino, 3-(2-Phenoxyethoxy)propylamino, 3-Benzyloxypropylamino, 2-Aminoethylamino, 2-(N,N-Dimethylamino)ethylamino, 2-(N,N-Diethylamino)ethylamino, 3-(N,N-Dimethylamino)propylamino, 3-(N,N-Diethylamino)propylamino, Benzylamino, 2-Phenylethylamino, 1-Phenylethylamino, Phenylamino, 2-Hydroxyphenylamino, 3-Hydroxyphenylamino, 4-Hydroxyphenylamino, 2-Methoxyphenylamino, 3-Methoxyphenylamino, 4-Methoxyphenylamino, 2-Ethoxyphenylamino, 3-Ethoxyphenylamino, 4-Ethoxyphenylamino, 2-Chlorphenylamino, 3-Chlorphenylamino, 4-Chlorphenylamino, 2-Nitrophenylamino, 3-Nitrophenylamino, 4-Nitrophenylamino, 2-Aminophenylamino, 3-Aminophenylamino, 4-Aminophenylamino, 2-Methylphenylamino, 3-Methylphenylamino, 4-Methylphenylamino, 2-Ethylphenylamino, 3-Ethylphenylamino, 4-Ethylphenylamino, Pyrid-2-ylamino, Pyrid-3-ylamino, Pyrid-4-ylamino, Methoxycarbonylmethylamino, Ethoxycarbonylmethylamino, Propoxycarbonylmethylamino, Butoxycarbonylmethylamino, 2-Methoxycarbonylmethylamino, 2-Ethoxycarbonylmethylamino, 2-Propoxycarbonylmethylamino, 2-Butoxycarbonylmethylamino, 2-(3,4-Dichlorphenyl)-ethylamino, 2-(3-Chlor-2-methylphenyl)ethylamino, 2-(3,4-Dimethoxyphenyl)ethylamino, 2-(2-Chlorphenyl)-ethylamino, Thien-2-ylamino, Dimethylamino, Diethylamino, Dipropylamino, Dibutylamino, Diallylamino, N-Methyl-N-butylamino, N-Methyl-N-phenylamino, N-Methyl-N-benzylamino, N-Ethyl-N-benzylamino, 3-Morpholinopropylamino, 2-(Piperazin-1-yl)ethylamino, Phenoxyethylamino, Phenoxypropylamino, 2-Methylthioethylamino, 3-Methylthiopropylamino, 2-Ethylthioethylamino, 3-Ethylthiopropylamino, 2-Aminoethylamino, 3-Aminopropylamino, 2-Dimethylaminoethylamino, 3-Dimethylaminopropylamino, 2-Diethylaminoethylamino, 3-Diethylaminopropylamino, Diethanolamino, N-Ethyl-N-phenylamino, Dibenzylamino, N-Methylpiperazino, 2-Pyrrolidinoethylamino, 2-Piperidinoethylamino, 2-Morpholinoethylamino, 2-Thiomorpholinoethylamino, 2-Hexamethyleniminoethylamino, 2-(N-Methylpiperazino)ethylamino, Hydrazino, N-Methylhydrazino, N-Ethylhydrazino, Phenylhydrazino, N,N-Dimethylhydrazino, N,N-Diethylhydrazino, N,N′-Dimethylhydrazino, N,N′-Diethylhydrazino, N,N-Dipropylhydrazino, N,N-Dibutylhydrazino, Imidazol-1-yl, Pyrazol-1-yl, 2,4-Dimethylphenylamino, 3-(Dimethylamino)phenylamino, 4-Cyanophenylamino, 2-Carboxyphenylamino, 4-Acetylphenylamino oder 2,4-Dichlorphenylamino.

Bevorzugt sind Diaminothiophene der Formel I, in der
$R^1$ und $R^2$ jeweils Wasserstoff oder zusammen den Rest

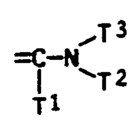,

worin $T^1$ für Wasserstoff, $C_1$-$C_4$-Alkyl oder Phenyl und $T^2$ und $T^3$ unabhängig voneinander für $C_1$-$C_4$-Alkyl oder Phenyl, oder zusammen mit dem sie verbindenden Stickstoffatom für Pyrrolidino, Piperidino, Morpholino, Thiomorpholino, Piperazino, N-($C_1$-$C_4$-Alkyl)piperazino oder Hexamethylenimino stehen, bedeuten und

$R^3$ und $R^4$ jeweils die obengenannte Bedeutung besitzen.

Besonders bevorzugt sind Diaminothiophene der Formel I, in der $R^1$ und $R^2$ jeweils Wasserstoff oder zusammen den Rest

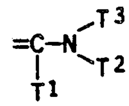

bedeuten, wobei $T^1$ für Wasserstoff und $T^2$ und $T^3$ unabhängig voneinander für $C_1$-$C_4$-Alkyl stehen.

Weiterhin besonders bevorzugt sind Diaminothiophene der Formel I, in der $R^4$ Formyl, Acetyl, Propionyl, Butyryl, Benzoyl, Cyano, Nitro oder einen Rest der Formel

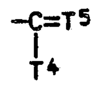

bedeutet, worin $T^4$ für Wasserstoff, Methyl, Ethyl oder Phenyl steht und $T^5$ die obengenannte Bedeutung besitzt.

Weiterhin besonders bevorzugt sind Diaminothiophene der Formel I, in der $R^3$ $C_1$-$C_6$-Mono- oder Dialkylamino, Allylamino, Methallylamino, Propargylamino, $C_2$-$C_6$-Alkylamino, das durch Phenyl, Hydroxy, Phenoxy, Amino oder $C_1$-$C_4$-Mono- oder Dialkylamino substituiert oder durch ein Sauerstoffatom unterbrochen ist, Phenylamino, Pyrrolidino, Piperidino, Morpholino, Piperazino oder N-($C_1$-$C_4$-Alkyl)piperazino bedeutet.

Weiterhin besonders bevorzugt sind Diaminothiophene der Formel I, in der $R^4$ Cyano, Nitro, Formyl oder den Rest

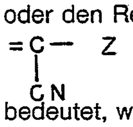

bedeutet, worin Z für Cyano, Nitro oder $C_1$-$C_4$-Alkoxycarbonyl steht.

Insbesondere von Bedeutung sind Diaminothiophene der Formel I, in der $R^1$ und $R^2$ jeweils Wasserstoff oder zusammen den Rest $=CH\text{-}N(CH_3)_2$ bedeuten.

Ganz besonders hervorzuheben sind Diaminothiophene der Formel Ia

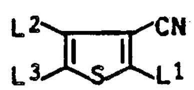

(Ia)

oder deren Tautomere, wobei

$L^1$ Amino,

$L^2$ $C_1$-$C_6$-Mono- oder Dialkylamino, Allylamino, Methallylamino, Propargylamino, $C_2$-$C_6$-Alkyl, das durch Phenyl, Hydroxy, Phenoxy, Amino oder $C_1$-$C_4$-Mono- oder Dialkylamino substituiert oder durch ein Sauerstoffatom unterbrochen ist, Phenylamino, Pyrrolidino, Piperidino, Morpholino, Piperazino oder N-($C_1$-$C_4$-Alkyl)piperazino und

$L^3$ Cyano, Nitro, Formyl oder den Rest

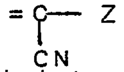

bedeuten, worin Z für Cyano, Nitro oder $C_1$-$C_4$-Alkoxycarbonyl steht.

Die erfindungsgemäßen Diaminothiophene der Formel I können z.B. durch Umsetzung von Aminothiophenen der Formel II

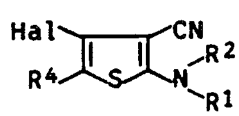

(II),

in der $R^1$, $R^2$ und $R^4$ jeweils die obengenannte Bedeutung besitzen und Hal für Chlor oder Brom steht, mit einer Aminoverbindung der Formel III

$R^3$-H (III),

in der $R^3$ die obengenannte Bedeutung besitzt, erhalten werden.

Dazu setzt man z.B. das Aminothiophen II mit der Aminoverbindung III in einem inerten Lösungsmittel (z.B. N,N-Dimethylformamid, N,N-Dimethylacetamid, N-Methylpyrrolidinon, Hexamethylphosphorsäuretrisamid, 1,3-Dimethylimidazolidin-2-on, 1,3-Dimethylhexahydropyrimidin-2-on oder 1,2-Diethoxyethan) bei einer Temperatur von -10 bis +150 °C um. Das Molverhältnis II:III beträgt dabei in der Regel 1:2 bis 1:10.

Die Aminothiophene der Formel II sind an sich bekannt und z.B. in der EP-A-193 895 beschrieben oder können analog den dort aufgeführten Methoden erhalten werden.

Die neuen Diaminothiophene sind wertvolle Zwischenprodukte für die Synthese von Farbstoffen, Pflanzenschutzmitteln oder Pharmazeutika. Insbesondere dienen sie als Diazokomponente ($R^1$, $R^2$ = H) für die Herstellung von Azofarbstoffen.

Die folgenden Beispiele sollen die Erfindung näher erläutern.

Beispiel 1

2-Amino-3,5-dicyano-4-piperidinothiophen

4,6 g 2-Amino-4-chlor-3,5-dicyanothiophen wurden in 25 ml N,N-Dimethylformamid (DMF) gelöst. Hierzu wurden bei Raumtemperatur 5,3 g Piperidin getropft und die Mischung wurde 4 Stunden bei Raumtemperatur gerührt. Anschließend wurde das Reaktionsgemisch in 800 g Wasser eingerührt. Der Niederschlag wurde abgesaugt, mit Wasser nachgewaschen und bei 50 °C im Trockenschrank getrocknet. Man erhielt 5,4 g (93 % d.Th.) der Verbindung der Formel

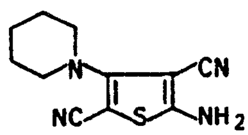

vom Schmelzpunkt 183 bis 184 °C. Das NMR-, IR-, UV- und Massenspektrum sowie die Elementaranalyse stimmen mit der oben angegebenen Konstitution überein.

In analoger Weise werden die in der folgenden Tabelle aufgeführten Verbindungen erhalten.

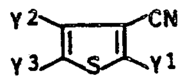

| Bsp.-Nr. | $Y^1$ | $Y^2$ | $Y^3$ | Schmelzpunkt [°C] |
|---|---|---|---|---|
| 2 | $NH_2$ | $NHCH_3$ | CN | |
| 3 | $NH_2$ | $NH-C_2H_5$ | CN | |
| 4 | $NH_2$ | $NH-n-C_3H_7$ | CN | 177-180 |
| 5 | $NH_2$ | $NH-iso-C_3H_7$ | CN | 170-171 |
| 6 | $NH_2$ | $NH-n-C_4H_9$ | CN | 211-212 |
| 7 | $NH_2$ | $NH-sec-C_4H_9$ | CN | 121-126 |
| 8 | $NH_2$ | $NH-iso-C_4H_9$ | CN | 178 |
| 9 | $NH_2$ | $NH-tert-C_4H_9$ | CN | 139-142 |
| 10 | $NH_2$ | $NH-n-C_5H_{11}$ | CN | 177-178 |
| 11 | $NH_2$ | $NH-iso-C_5H_{11}$ | CN | |
| 12 | $NH_2$ | $NH-neo-C_5H_{11}$ | CN | |
| 13 | $NH_2$ | $NH-n-C_6H_{13}$ | CN | |
| 14 | $NH_2$ | $NH-n-C_7H_{15}$ | CN | 92- 97 |
| 15 | $NH_2$ | $NH-n-C_8H_{17}$ | CN | |
| 16 | $NH_2$ | $NH-n-C_9H_{19}$ | CN | |
| 17 | $NH_2$ | $NH-n-C_{10}H_{21}$ | CN | |
| 18 | $NH_2$ | $NH-n-C_{11}H_{23}$ | CN | |
| 19 | $NH_2$ | $NH-n-C_{12}H_{25}$ | CN | |
| 20 | $NH_2$ | $NH-n-C_{13}H_{27}$ | CN | |
| 21 | $NH_2$ | $NH-n-C_{14}H_{29}$ | CN | |
| 22 | $NH_2$ | $NH-n-C_{15}H_{31}$ | CN | |
| 23 | $NH_2$ | $NH-n-C_{16}H_{33}$ | CN | |
| 24 | $NH_2$ | $NH-n-C_{17}H_{35}$ | CN | |
| 25 | $NH_2$ | $NH-n-C_{18}H_{37}$ | CN | |
| 26 | $NH_2$ | $NH-n-C_{19}H_{39}$ | CN | |
| 27 | $NH_2$ | $NH-n-C_{20}H_{41}$ | CN | |
| 28 | $NH_2$ | $NH-CH_2-CH=CH_2$ | CN | |
| 29 | $NH_2$ | $NH-CH_2-C\equiv CH$ | CN | |
| 30 | $NH_2$ | $NH-CH_2-CH=CH-CH_3$ | CN | |
| 31 | $NH_2$ | $NH-CH_2-CH_2-OH$ | CN | 170 |
| 32 | $NH_2$ | $NH-CH_2-CH_2-OCH_3$ | CN | |
| 33 | $NH_2$ | $NH-CH_2-CH_2-OC_2H_5$ | CN | 175 |

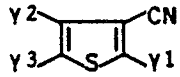

| Bsp.-Nr. | $Y^1$ | $Y^2$ | $Y^3$ | Schmelzpunkt [°C] |
|---|---|---|---|---|
| 34 | $NH_2$ | $NH-CH_2-CH_2-O-nC_3H_7$ | CN | |
| 35 | $NH_2$ | $NH-CH_2-CH_2-OC_6H_5$ | CN | |
| 36 | $NH_2$ | $NH-CH_2-CH_2-OCH_2-C_6H_5$ | CN | |
| 37 | $NH_2$ | $NH-CH_2-CH_2-NH_2$ | CN | |
| 38 | $NH_2$ | $NH-CH_2-CH_2-N(CH_3)_2$ | CN | |
| 39 | $NH_2$ | $NH-CH_2-CH_2-N(C_2H_5)_2$ | CN | 125-129 |
| 40 | $NH_2$ | $NH-(CH_2)_3-OH$ | CN | 171-172 |
| 41 | $NH_2$ | $NH-(CH_2)_3-OCH_3$ | CN | |
| 42 | $NH_2$ | $NH-(CH_2)_3-OC_2H_5$ | CN | |
| 43 | $NH_2$ | $NH-(CH_2)_3-O-n-C_3H_7$ | CN | |
| 44 | $NH_2$ | $NH-(CH_2)_3-OC_6H_5$ | CN | |
| 45 | $NH_2$ | $NH-(CH_2)_3-OCH_2C_6H_5$ | CN | |
| 46 | $NH_2$ | $NH-(CH_2)_3-O(CH_2)_2-OC_6H_5$ | CN | |
| 47 | $NH_2$ | $NH-(CH_2)_3-NH_2$ | CN | |
| 48 | $NH_2$ | $NH-(CH_2)_3-N(CH_3)_2$ | CN | |
| 49 | $NH_2$ | $NH-(CH_2)_3-N(C_2H_5)_2$ | CN | |
| 50 | $NH_2$ | $NH-C_6H_5$ | CN | |
| 51 | $NH_2$ | $NH-CH_2C_6H_5$ | CN | |
| 52 | $NH_2$ | $NH-CH_2CH_2C_6H_5$ | CN | |
| 53 | $NH_2$ | $NH-CH_2(CH_3)C_6H_5$ | CN | |
| 54 | $NH_2$ | $NH-cyclo-C_3H_5$ | CN | |
| 55 | $NH_2$ | $NH-cyclo-C_4H_7$ | CN | |
| 56 | $NH_2$ | $NH-cyclo-C_5H_9$ | CN | |
| 57 | $NH_2$ | $NH-cyclo-C_6H_{11}$ | CN | |
| 58 | $NH_2$ | $NH-cyclo-C_7H_{13}$ | CN | |
| 59 | $NH_2$ | $NH-cyclo-C_8H_{15}$ | CN | |
| 60 | $NH_2$ | $NH-CH_2-CH_2-SCH_3$ | CN | |
| 61 | $NH_2$ | $NH-CH_2-CH_2-SC_2H_5$ | CN | |
| 62 | $NH_2$ | $NH-(CH_2)_3SCH_3$ | CN | |
| 63 | $NH_2$ | $NH-(CH_2)_3SC_2H_5$ | CN | |
| 64 | $NH_2$ | $NH-C_6H_4-(2)-CH_3$ | CN | |
| 65 | $NH_2$ | $NH-C_6H_4-(3)-CH_3$ | CN | |

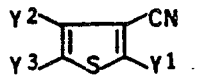

| Bsp.-Nr. | Y1 | Y2 | Y3 | Schmelzpunkt [°C] |
|---|---|---|---|---|
| 66 | $NH_2$ | $NH-C_6H_4-(4)-CH_3$ | CN | |
| 67 | $NH_2$ | $NH-C_6H_4-(2)-C_2H_5$ | CN | |
| 68 | $NH_2$ | $NH-C_6H_4-(3)-C_2H_5$ | CN | |
| 69 | $NH_2$ | $NH-C_6H_4-(4)-C_2H_5$ | CN | |
| 70 | $NH_2$ | $NH-C_6H_4-(2)-OH$ | CN | |
| 71 | $NH_2$ | $NH-C_6H_4-(3)-OH$ | CN | |
| 72 | $NH_2$ | $NH-C_6H_4-(4)-OH$ | CN | |
| 73 | $NH_2$ | $NH-C_6H_4-(2)-Cl$ | CN | |
| 74 | $NH_2$ | $NH-C_6H_4-(3)-Cl$ | CN | |
| 75 | $NH_2$ | $NH-C_6H_4-(4)-Cl$ | CN | |
| 76 | $NH_2$ | $NH-C_6H_4-(2)-OCH_3$ | CN | |
| 77 | $NH_2$ | $NH-C_6H_4-(3)-OCH_3$ | CN | |
| 78 | $NH_2$ | $NH-C_6H_4-(4)-OCH_3$ | CN | |
| 79 | $NH_2$ | $NH-C_6H_4-(2)-OC_2H_5$ | CN | |
| 80 | $NH_2$ | $NH-C_6H_4-(3)-OC_2H_5$ | CN | |
| 81 | $NH_2$ | $NH-C_6H_4-(4)-OC_2H_5$ | CN | |
| 82 | $NH_2$ | $NH-C_6H_4-(2)-NH_2$ | CN | |
| 83 | $NH_2$ | $NH-C_6H_4-(3)-NH_2$ | CN | |
| 84 | $NH_2$ | $NH-C_6H_4-(4)-NH_2$ | CN | |
| 85 | $NH_2$ | $NH-C_6H_3-(2,3)-(CH_3)_2$ | CN | |
| 86 | $NH_2$ | $NH-C_6H_3-(2,4)-(CH_3)_2$ | CN | |
| 87 | $NH_2$ | $NH-C_6H_3-(2,5)-(CH_3)_2$ | CN | |
| 88 | $NH_2$ | $NH-C_6H_3-(2,6)-(CH_3)_2$ | CN | |
| 89 | $NH_2$ | $NH-C_6H_3-(3,4)-(CH_3)_2$ | CN | |
| 90 | $NH_2$ | $NH-C_6H_3-(3,5)-(CH_3)_2$ | CN | |
| 91 | $NH_2$ | $NH-C_6H_4-(2)-N(CH_3)_2$ | CN | |
| 92 | $NH_2$ | $NH-C_6H_4-(3)-N(CH_3)_2$ | CN | |
| 93 | $NH_2$ | $NH-C_6H_4-(4)-N(CH_3)_2$ | CN | |
| 94 | $NH_2$ | $NH-C_6H_4-(2)-CN$ | CN | |
| 95 | $NH_2$ | $NH-C_6H_4-(3)-CN$ | CN | |
| 96 | $NH_2$ | $NH-C_6H_4-(4)-CN$ | CN | |
| 97 | $NH_2$ | $NH-C_6H_4-(2)-COOH$ | CN | |

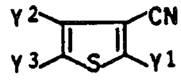

| Bsp.-Nr. | Y1 | Y2 | Y3 | Schmelzpunkt [°C] |
|---|---|---|---|---|
| 98 | $NH_2$ | $NH-C_6H_4-(3)-COOH$ | CN | |
| 99 | $NH_2$ | $NH-C_6H_4-(4)-COOH$ | CN | |
| 100 | $NH_2$ | $NH-C_6H_4-(2)-CO-CH_3$ | CN | |
| 101 | $NH_2$ | $NH-C_6H_4-(3)-CO-CH_3$ | CN | |
| 102 | $NH_2$ | $NH-C_6H_4-(4)-CO-CH_3$ | CN | |
| 103 | $NH_2$ | $NH-C_6H_4-(2,3)-Cl_2$ | CN | |
| 104 | $NH_2$ | NH-pyrid-2-yl | CN | |
| 105 | $NH_2$ | NH-pyrid-3-yl | CN | |
| 106 | $NH_2$ | NH-pyrid-4-yl | CN | |
| 107 | $NH_2$ | NH-thien-2-yl | CN | |
| 108 | $NH_2$ | $N(CH_3)_2$ | CN | |
| 109 | $NH_2$ | $N(C_2H_5)_2$ | CN | 142-144 |
| 110 | $NH_2$ | $N(n-C_3H_7)_2$ | CN | |
| 111 | $NH_2$ | $N(n-C_4H_9)_2$ | CN | |
| 112 | $NH_2$ | $N(CH_2CH{=}CH_2)_2$ | CN | |
| 113 | $NH_2$ | $N(CH_3)(C_6H_5)$ | CN | |
| 114 | $NH_2$ | $N(C_2H_5)(CH_2C_6H_5)$ | CN | |
| 115 | $NH_2$ | $N(CH_3)(C_6H_5)$ | CN | |
| 116 | $NH_2$ | $N(C_2H_5)(CH_2C_6H_5)$ | CN | |
| 117 | $NH_2$ | $N(CH_2C_6H_5)_2$ | CN | |
| 118 | $NH_2$ | $N(CH_2CH_2OH)_2$ | CN | |
| 119 | $NH_2$ | 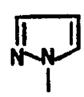 | CN | |
| 120 | $NH_2$ | 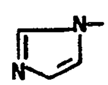 | CN | |
| 121 | $NH_2$ | 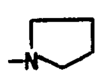 | CN | 302-303 |
| 122 | $NH_2$ | 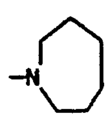 | CN | 137-138 |

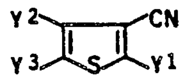

| Bsp.-Nr. | Y1 | Y2 | Y3 | Schmelzpunkt [°C] |
|---|---|---|---|---|
| 123 | $NH_2$ | 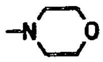 | CN | |
| 124 | $NH_2$ | 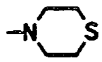 | CN | |
| 125 | $NH_2$ | 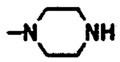 | CN | |
| 126 | $NH_2$ | 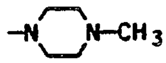 | CN | |
| 127 | $NH_2$ | 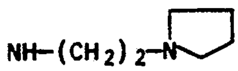 | CN | |
| 128 | $NH_2$ | 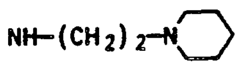 | CN | |
| 129 | $NH_2$ | 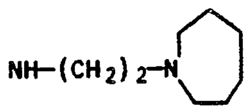 | CN | |
| 130 | $NH_2$ | 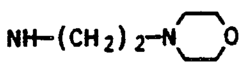 | CN | |
| 131 | $NH_2$ | 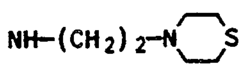 | CN | |
| 132 | $NH_2$ | 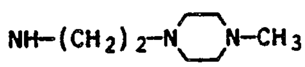 | CN | |
| 133 | $NH_2$ | 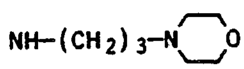 | CN | |
| 134 | $NH_2$ | $NH-NH_2$ | CN | |
| 135 | $NH_2$ | $NH-N(CH_3)_2$ | CN | |
| 136 | $NH_2$ | $NH-NH-C_6H_5$ | CN | |
| 137 | $NH_2$ | $NH-NH-CH_3$ | CN | |
| 138 | $NH_2$ | $N(CH_3)-NH-CH_3$ | CN | |
| 139 | $NH_2$ | $NH-N(C_2H_5)_2$ | CN | |
| 140 | $NH_2$ | $NH-N(n-C_3H_7)_2$ | CN | |
| 141 | $NH_2$ | $NH-N(n-C_4H_9)_2$ | CN | |

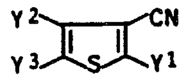

| Bsp.-Nr. | Y1 | Y2 | Y3 | Schmelzpunkt [°C] |
|---|---|---|---|---|
| 142 | $N{=}CH{-}N(CH_3)_2$ | $N(C_2H_5)_2$ | CN | |
| 143 | $N{=}CH{-}N(CH_3)_2$ | $N(CH_3)_2$ | CN | |
| 144 | $N{=}CH{-}N(C_2H_5)_2$ | $N(CH_3)_2$ | CN | |
| 145 | $N{=}CH{-}N(C_2H_5)_2$ | $N(C_2H_5)_2$ | CN | |
| 146 | $NH_2$ | $NH{-}CH_3$ | $NO_2$ | |
| 147 | $NH_2$ | $NH{-}C_2H_5$ | $NO_2$ | |
| 148 | $NH_2$ | $NH{-}n{-}C_3H_7$ | $NO_2$ | |
| 149 | $NH_2$ | $NH{-}iso{-}C_3H_7$ | $NO_2$ | |
| 150 | $NH_2$ | $NH{-}n{-}C_4H_9$ | $NO_2$ | |
| 151 | $NH_2$ | $NH{-}n{-}C_5H_{11}$ | $NO_2$ | |
| 152 | $NH_2$ | $NH{-}cyclo{-}C_3H_5$ | $NO_2$ | |
| 153 | $NH_2$ | $NH{-}cyclo{-}C_6H_{11}$ | $NO_2$ | |
| 154 | $NH_2$ | 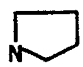 | $NO_2$ | > 315 |
| 155 | $NH_2$ | 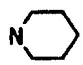 | $NO_2$ | |
| 156 | $NH_2$ | 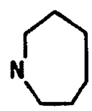 | $NO_2$ | |
| 157 | $NH_2$ | 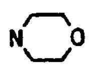 | $NO_2$ | 275-276 |
| 158 | $NH_2$ | 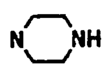 | $NO_2$ | |
| 159 | $NH_2$ | $NH{-}CH_3$ | $CH{=}N{-}CH_3$ | |
| 160 | $NH_2$ | $NH{-}C_2H_5$ | $CH{=}N{-}C_2H_5$ | |
| 161 | $NH_2$ | $N(CH_3)_2$ | CHO | |
| 162 | $NH_2$ | $N(C_2H_5)_2$ | CHO | |
| 163 | $NH_2$ | $N(n{-}C_3H_7)_2$ | CHO | |
| 164 | $NH_2$ | $N(n{-}C_4H_9)_2$ | CHO | |
| 165 | $NH_2$ | 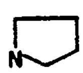 | CHO | |

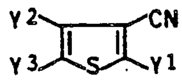

| Bsp.-Nr. | $Y^1$ | $Y^2$ | $Y^3$ | Schmelzpunkt [°C] |
|---|---|---|---|---|
| 166 | $NH_2$ | 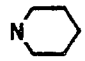 | CHO | |
| 167 | $NH_2$ | 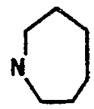 | CHO | |
| 168 | $NH_2$ | 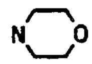 | CHO | |
| 169 | $NH_2$ | 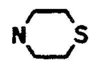 | CHO | |
| 170 | $NH_2$ | 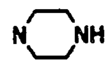 | CHO | |
| 171 | $NH_2$ | 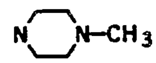 | CHO | |
| 172 | $NH_2$ | $N(CH_3)_2$ | $CO-CH_3$ | |
| 173 | $NH_2$ | $N(C_2H_5)_2$ | $CO-CH_3$ | |
| 174 | $NH_2$ | $N(CH_3)_2$ | $CO-C_2H_5$ | |
| 175 | $NH_2$ | $N(C_2H_5)_2$ | $CO-n-C_3H_7$ | |
| 176 | $NH_2$ | $N(CH_3)_2$ | $CO-iso-C_3H_7$ | |
| 177 | $NH_2$ | $N(CH_3)_2$ | $CO-C_6H_5$ | |
| 178 | $NH_2$ | $N(CH_3)_2$ | $CH=C(CN)_2$ | |
| 179 | $NH_2$ | $N(C_2H_5)_2$ | $CH=C(CN)_2$ | 107-109 |
| 180 | $NH_2$ | $N(n-C_3H_7)_2$ | $CH=C(CN)_2$ | |
| 181 | $NH_2$ | $N(n-C_4H_9)_2$ | $CH=C(CN)_2$ | |
| 182 | $NH_2$ | 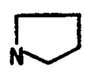 | $CH=C(CN)_2$ | |
| 183 | $NH_2$ | 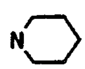 | $CH=C(CN)_2$ | 225 |
| 184 | $NH_2$ | 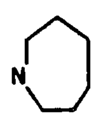 | $CH=C(CN)_2$ | |
| 185 | $NH_2$ | 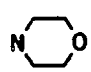 | $CH=C(CN)_2$ | 270 |

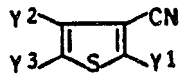

| Bsp.-Nr. | Y1 | Y2 | Y3 | Schmelzpunkt [°C] |
|---|---|---|---|---|
| 186 | $NH_2$ | $NH-CH(C_6H_5)_2$ | 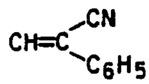 | |
| 187 | $NH_2$ | $NH-CH_3$ | 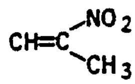 | |
| 188 | $NH_2$ | $NH-C_2H_5$ | 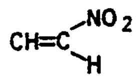 | |
| 189 | $NH_2$ | 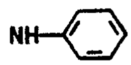 | 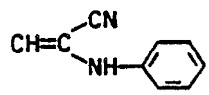 | |
| 190 | $NH_2$ | $N(C_2H_5)_2$ | 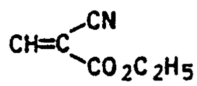 | |
| 191 | $NH_2$ | 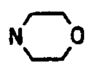 | 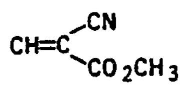 | |
| 192 | $NH_2$ | $NH-CH_3$ | 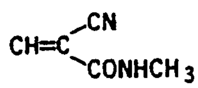 | |
| 193 | $NH_2$ | $NH-C_2H_5$ | 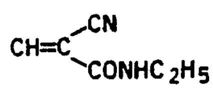 | |
| 194 | $NH_2$ | 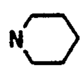 | 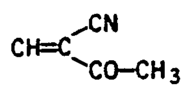 | |
| 195 | $NH_2$ | 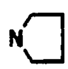 | 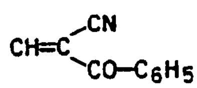 | |
| 196 | $NH_2$ | 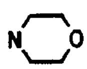 | 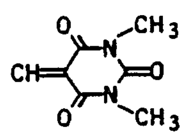 | |
| 197 | $NH_2$ | $NHCH_3$ | 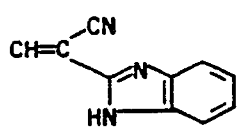 | |
| 198 | $NH_2$ | $NHC_2H_5$ | $CH=C(CO_2CH_3)_2$ | |
| 199 | $NH_2$ | $N(CH_3)_2$ | 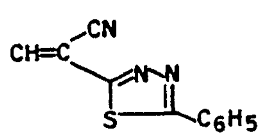 | |
| 200 | $NH_2$ | $NH-C_6H_5$ | 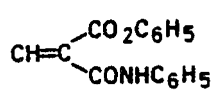 | |

## Ansprüche

1. Diaminothiophene der Formel I

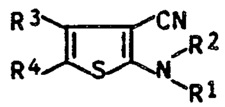

(I)

oder deren Tautomere, wobei
$R^1$ und $R^2$ jeweils Wasserstoff oder zusammen einen Rest der Formel

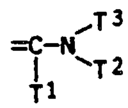

,

worin $T^1$ für Wasserstoff, $C_1$-$C_4$-Alkyl oder Phenyl und $T^2$ und $T^3$ gleich oder verschieden sind und unabhängig voneinander jeweils für $C_1$-$C_4$-Alkyl, $C_5$-$C_7$-Cycloalkyl oder Phenyl oder $T^2$ und $T^3$ zusammen mit dem sie verbindenden Stickstoffatom für einen 5- bis 7-gliedrigen gesättigten heterocyclischen Rest, der gegebenenfalls weitere Heteroatome enthält, stehen,
$R^3$ $C_1$-$C_{20}$-Mono- oder Dialkylamino, $C_2$-$C_{10}$-Mono- oder Dialkylamino, worin die Alkylkette substituiert und/oder durch ein oder mehrere Sauerstoffatome unterbrochen ist, $C_3$-$C_8$-Cycloalkylamino, Adamantylamino, $C_2$-$C_{12}$-Mono- oder Dialkenylamino, $C_3$-$C_{12}$-Alkinylamino, N-($C_1$-$C_5$-Alkyl)-N-phenylamino, Pyrrolidino, Piperidino, Morpholino, Thiomorpholino, Piperazino, N-($C_1$-$C_4$-Alkyl)piperazino, Hexamethylenimino, Imidazol-1-yl, Pyrazol-1-yl, gegebenenfalls substituiertes Phenylamino, Pyridylamino, Thienylamino, Hydrazino, $C_1$-$C_4$-Mono- oder Dialkylhydrazino oder Phenylhydrazino und
$R^4$ $C_1$-$C_6$-Alkanoyl, Benzoyl, Cyano, Nitro oder einen Rest der Formel

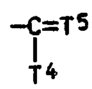

bedeuten, worin $T^4$ für Wasserstoff, $C_1$-$C_4$-Alkyl oder Phenyl und $T^5$ für den Rest einer methylenaktiven Verbindung, Hydroxyimino oder den Rest N-X stehen, in dem X die Bedeutung von $C_1$-$C_{20}$-Alkyl, das gegebenenfalls substituiert und/oder durch ein oder mehrere Sauerstoffatome unterbrochen ist, gegebenenfalls substituiertem $C_3$-$C_6$-Alkenyl, gegebenenfalls substituiertem $C_3$-$C_6$-Alkinyl, gegebenenfalls substituiertem $C_3$-$C_{10}$-Cycloalkyl, gegebenenfalls substituiertem Phenyl, Pyridyl, $C_1$-$C_4$-Alkoxycarbonylmethyl, Amino, $C_1$-$C_4$-Dialkylamino oder Phenylamino besitzt.

2. Diaminothiophene gemäß Anspruch 1, dadurch gekennzeichnet, daß
$R^1$ und $R^2$ jeweils Wasserstoff oder zusammen den Rest

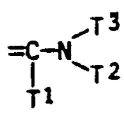

,

worin $T^1$ für Wasserstoff, $C_1$-$C_4$-Alkyl oder Phenyl und $T^2$ und $T^3$ unabhängig voneinander für $C_1$-$C_4$-Alkyl oder Phenyl, oder zusammen mit dem sie verbindenden Stickstoffatom für Pyrrolidino, Piperidino, Morpholino, Thiomorpholino, Piperazino, N-($C_1$-$C_4$-Alkyl)piperazino oder Hexamethylenimino stehen, bedeuten und $R^3$ und $R^4$ jeweils die in Anspruch 1 genannte Bedeutung besitzen.

3. Verfahren zur Herstellung von Diaminothiophenen gemäß Anspruch 1, dadurch gekennzeichnet, daß man Aminothiophene der Formel II

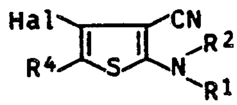

(II),

in der $R^1$, $R^2$ und $R^4$ jeweils die in Anspruch 1 genannte Bedeutung besitzen und Hal für Chlor oder Brom steht, mit einer Aminoverbindung der Formel III

$R^3$-H (III),

in der $R^3$ die in Anspruch 1 genannte Bedeutung besitzt, umsetzt.